# EUROPÄISCHE PATENTANMELDUNG

(11) **EP 4 035 722 A1**
(43) Veröffentlichungstag der Anmeldung: **03.08.2022**
(21) Anmeldenummer: 21153806.1
(22) Anmeldetag: 27.01.2021
(51) Int. Cl.: A61M 35/00, A61F 11/00

(54) **GERÄT ZUR ABGABE EINES WIRKSTOFFS INS OHRINNERE**

(71) Anmelder: Sauder, Yusuf, 8580 Amriswil (CH)
(72) Erfinder: Sauder, Yusuf, 8580 Amriswil (CH)
(74) Vertreter: Kaminski Harmann Patentanwälte AG (Schweiz)

(57) **Zusammenfassung**

Die Erfindung betrifft ein Gerät, das am oder im Ohr tragbar und zur Abgabe eines Wirkstoffs ins Ohrinnere ausgelegt ist. Dazu weist das Gerät eine Energieversorgung, eine Schnittstelle zum Empfangen von Steueranweisungen, ein Gerätegehäuse, eine Aufnahme für ein Wirkstoffpräparat, eine Aufbereitungseinheit, und einen Stoffausgang auf. Die Aufbereitungseinheit wird mittels der Schnittstelle gesteuert und ist dazu ausgelegt, ein Wirkstoffpräparat so aufzubereiten, dass der Wirkstoff in transportfähiger Form vorliegt und über den Stoffausgang ins Ohrinnere abgebbar ist. Die Energieversorgung, die Schnittstelle, die Aufnahme, die Aufbereitungseinheit und der Stoffausgang bilden mittels des Gerätegehäuses eine Einheit.

## Beschreibung

### -FACHGEBIET DER ERFINDUNG-

Die Erfindung betrifft ein am oder im Ohr tragbares Gerät, das zur Abgabe eines Wirkstoffs ins Ohrinnere ausgelegt ist.

### -HINTERGRUND-

Beschwerden die im Zusammenhang mit einem Zustand des Ohrinneren auftreten sind für die Betroffenen unangenehm und verlangen in den meisten Fällen nach einer Behandlung. Solche Beschwerden reichen von einem einfachen Juckreiz bis hin zu starken Ohrenschmerzen. Aufgrund solcher Beschwerden, suchen Betroffene medizinische Hilfe, beispielsweise bei einem Hals-Nasen-Ohrenarzt, mit dem Ziel die Ursache für die Beschwerden zu finden und eine entsprechende Behandlung zur Bekämpfung der Ursache zu erhalten. Die möglichen Ursachen sind zahlreich und können unterschiedlichster Natur sein. Oft ist die Ursache jedoch eine Entzündung eines Teils der Haut, die die Wand bzw. die Oberfläche des Ohrinneren mitbildet. Eine solche Entzündung steht häufig im Zusammenhang mit einer bakteriellen Infektion, kann aber auch im Zusammenhang mit einer viralen, pilzbasierten oder nichtspezifischen Infektion stehen. In den meisten Fällen in denen eine derartige Entzündung vorliegt kann die Ursache wirksam bekämpft werden, indem eine Behandlung der Haut im Ohrinneren mit einem Wirkstoff, der dazu ins Ohrinnere eingebracht wird, erfolgt.

Die Anwendung von Ohrentropfen die den Wirkstoff enthalten ist wohl eine der am weitest verbreiteten Behandlungsvarianten, um solche Ursachen zu bekämpfen. Dazu legt sich der Betroffene mit dem zu behandelnden Ohr nach oben auf die Seite und tropft die Lösung die den Wirkstoff enthält ins Ohrinnere ein. Die seitliche Lage ist dann für eine gewisse Zeitdauer, typischerweise 15 Minuten, beizubehalten, damit die Lösung und der Wirkstoff auf die Haut im Ohrinnern einwirken können. Anschliessend wird das Ohr mit Watte verschlossen.

Eine alternative Behandlungsvariante ist in der Patentschrift DE 2145333 A1 offenbart. Dabei wird ein Gehörgangdocht bereitgestellt der zu Behandlungszwecken in den Gehörgang eingesetzt werden kann. In eingesetztem Zustand wird dann die Behandlungsflüssigkeit eingeträufelt und vom Gehörgangdocht aufgenommen. Der Gehörgangdocht füllt dann den gesamten äusseren Gehörgang aus, sodass die Behandlungsflüssigkeit in Berührung mit der Wandung des Gehörgangs gehalten wird.

Die Schrift CN 110037856 A offenbart eine weitere alternative Vorrichtung, die zur Behandlung des Ohrinneren mit einem Wirkstoff durch einen Spezialisten ausgelegt ist.

Es ist Aufgabe der vorliegenden Erfindung ein flexibel einsetzbares und einfach zu bedienendes Gerät bereitzustellen, welches dazu ausgelegt ist ein Wirkstoff zur Behandlung des Ohrinneren ins Ohrinnere abzugeben.

Es ist eine weitere Aufgabe der Erfindung, ein Gerät bereitzustellen, das von einem Benutzer ohne spezifische medizinische Fachkenntnisse benutzt werden kann. Dies vor allem im Hinblick darauf, dass chronische Beschwerden im Zusammenhang mit einem Zustand des Ohrinneren einfach behandelbar sind.

Es ist eine weitere Aufgabe der Erfindung ein Gerät bereitzustellen, welches den Benutzer bei der Benutzung nicht unnötig einschränkt bzw. seine akustische Wahrnehmung nur möglichst kurzzeitig beeinträchtigt.

### -BESCHREIBUNG DER ERFINDUNG-

Die Erfindung betrifft ein Gerät, das am oder im Ohr tragbar und zur Abgabe eines Wirkstoffs ins Ohrinnere bzw. zur Applikation eines Wirkstoffs im Ohrinneren ausgelegt ist. Dazu weist das Gerät eine Energieversorgung, eine Schnittstelle zum Empfangen von Steueranweisungen, ein Gerätegehäuse, eine Aufnahme für ein Wirkstoffpräparat, eine Aufbereitungseinheit, und einen Stoffausgang auf. Die Aufbereitungseinheit ist mittels der Schnittstelle steuerbar und ist dazu ausgelegt, ein, insbesondere aufgenommenes, Wirkstoffpräparat so aufzubereiten, dass der Wirkstoff in transportfähiger Form vorliegt und über den Stoffausgang ins Ohrinnere abgebbar bzw. im Ohrinneren applizierbar ist. Dabei bildet die Energieversorgung, die Schnittstelle, die Aufnahme, die Aufbereitungseinheit und der Stoffausgang mittels des Gerätegehäuses eine Einheit.

Das Gerät kann so ausgestaltet sein, dass es in einer mit einem Hinter-dem-Ohr-Hörgerät vergleichbaren Weise am Ohr tragbar ist. Dazu kann es beispielsweise entsprechend ergonomisch geformt sein, sodass es auf die Ohrmuschel aufgesetzt werden kann.

Das Gerät kann auch so ausgestaltet sein, dass es in einer mit einem Im-Ohr-Hörgerät vergleichbaren Weise im Ohr tragbar ist. Dazu kann es beispielsweise entsprechend kompakt ausgebildet sein, sodass es beispielsweise im Gehörgang positioniert werden kann.

Diese Art von Tragbarkeit ermöglicht einem Benutzer eine flexible Nutzung des Geräts, sodass dieser in seiner Bewegungsfreiheit kaum eingeschränkt ist.

Das Gerät ist erfindungsgemäss dazu ausgelegt eine Transportverbindung zum Ohrinneren bereitzustellen. Über diese Transportverbindung kann eine Verabreichung eines Wirkstoffs ins Ohrinnere bzw. eine Applikation eines Wirkstoffs im Ohrinneren erfolgen. Die Applikation des Wirkstoffes kann also intraaurikulär erfolgen. Die Transportverbindung ermöglicht dabei den gezielten Transport des Wirkstoffs, der in transportfähiger Form vorliegt, von dem Gerät zum Inneren des Ohrs zu der Stelle an der die Behandlung erfolgen soll. Die transportfähige Form kann dabei beispielsweise gasförmig, dampfförmig, pulverförmig, flüssig oder eine Mischung davon sein, je nachdem in welcher Form das Wirkstoffpräparat vorliegt. Das Ohrinnere umfasst die Bereiche die sich vom Aussenohr nach Innen erstrecken und somit beispielsweise den Gehörgang, das Mittelohr und das Innenohr.

Das Gerät hat eine Energieversorgung, die beispielsweise über einen Energiespeicher bereitgestellt wird. Mögliche Energiespeicher können Batterien, insbesondere wiederaufladbare Batterien, und/oder Kondensatoren sein.

Das Gerät hat eine Schnittstelle zum Empfangen von Steueranweisungen und eine Aufbereitungseinheit. Die Schnittstelle und die Aufbereitungseinheit werden von der Energieversorgung gespeist. Die Schnittstelle kann die Steueranweisungen über eine kommunikative Verbindung zu einer Steuerung von dieser Steuerung erhalten. Die Steuerung kann Teil des Geräts sein oder auch nicht. Beispielsweise kann die Steuerung auch über eine Recheneinheit eines mobilen Datenverarbeitungsgeräts, wie beispielsweise eines Mobiltelefons, Tablet-Computers, Laptops etc., das nicht Teil des Geräts ist bereitgestellt sein. Die kommunikative Verbindung mit der Steuerung kann beispielsweise auf eine kabelgebundene oder auch kabellose Art bereitgestellt sein. Die Schnittstelle ist kommunikativ mit der Aufbereitungseinheit verbunden, sodass die Aufbereitungseinheit über die Schnittstelle kontrolliert/gesteuert werden kann. Diese kommunikative Verbindung kann beispielsweise auf eine kabelgebundene oder auch kabellose Art bereitgestellt sein.

Das Gerät hat eine Aufnahme die dazu ausgelegt ist ein Wirkstoffpräparat aufzunehmen. Die Aufnahme kann so ausgestaltet sein, dass das Wirkstoffpräparat beispielsweise in die Aufnahme eingelegt, eingesetzt oder eingefüllt wird. Es kann auch vorgesehen sein, dass die Aufnahme eine verschliessbare Öffnung bildet durch die das Wirkstoffpräparat aufgenommen und welche nach Aufnahme des Wirkstoffpräparats geschlossen wird.

Das Wirkstoffpräparat enthält den Wirkstoff und kann beispielsweise ein Pulver, eine Flüssigkeit, eine Paste, ein Gel oder eine Creme sein. Das Wirkstoffpräparat kann auch in Form einer Kapsel bereitgestellt sein. Dabei hat die Kapsel eine Kapselhülle und eine, durch die Kapselhülle gegen aussen abgeschlossene, Füllung. Typischerweise enthält die Füllung der Kapsel den Wirkstoff. Die Füllung der Kapsel kann ein Pulver, eine Flüssigkeit, eine Paste, eine Creme, ein Gel oder auch gasförmig sein.

Die Aufbereitungseinheit des Geräts ist dazu ausgelegt das Wirkstoffpräparat, das sich in der Aufnahme befindet, aufzubereiten. Dazu erhält die Aufbereitungseinheit von der Steuerung über die Schnittstelle Anweisungen und ist durch diese auch kontrollierbar. Das Gerät kann auch eine Eingabevorrichtung aufweisen, wobei dann die Schnittstelle kommunikativ mit der Eingabevorrichtung verbunden ist. Diese kommunikative Verbindung kann beispielsweise auf eine kabelgebundene oder kabellose Art bereitgestellt sein. Die Aufbereitungseinheit kann dann von einem Benutzer durch Betätigung der Eingabevorrichtung angewiesen werden die Aufbereitung zu veranlassen. Die Veranlassung kann aber auch durch beliebige andere Trigger-Mechanismen ausgelöst werden, wie beispielsweise das Schliessen der Öffnung der Aufnahme etc. Die Aufbereitung kann folgendes umfassen:

Ist das Wirkstoffpräparat ein Pulver, wird dieses soweit erwärmt bis der im Pulver enthaltene Wirkstoff siedet/verdampft/in den gasförmigen Zustand übergeht, sodass der gasförmige und/oder dampfförmige Anteil des Pulvers die transportfähige Form bildet und über den Stoffausgang des Geräts ins Ohrinnere abgegeben wird. Das Pulver kann im Rahmen der Aufbereitung auch verwirbelt werden, sodass Pulverteilchen die transportfähige Form bilden, beispielsweise in der Form eines Aerosols, und über den Stoffausgang des Geräts ins Ohrinnere abgegeben werden.

Ist das Wirkstoffpräparat eine Flüssigkeit, wird diese soweit erwärmt bis der Wirkstoff siedet/verdampft/in den gasförmigen Zustand übergeht, sodass der gasförmige und/oder dampfförmige Anteil der Flüssigkeit die transportfähige Form bildet und über den Stoffausgang des Geräts ins Ohrinnere abgegeben wird.

Ist das Wirkstoffpräparat eine Flüssigkeit, wird diese soweit erwärmt, dass aufgrund der thermischen Ausdehnung der Flüssigkeit, diese als Flüssigkeit über den Stoffausgang des Geräts zum Ohrinneren transportiert wird und ins Ohrinnere abgegeben wird. Es kann vorgesehen sein, dass die Flüssigkeit zur Abgabe ins Ohrinnere zerstäubt wird, beispielsweise mittels eines Zerstäubers.

Ist das Wirkstoffpräparat eine Paste, wird diese soweit erwärmt bis der in der Paste enthaltene Wirkstoff siedet/verdampft/in den gasförmigen Zustand übergeht, sodass der gasförmige und/oder dampfförmige Anteil der Paste die transportfähige Form bildet und über den Stoffausgang des Geräts ins Ohrinnere abgegeben wird.

Ist das Wirkstoffpräparat eine Creme oder ein Gel wird diese/s soweit erwärmt bis der in der Creme oder dem Gel enthaltene Wirkstoff siedet/verdampft/in den gasförmigen Zustand übergeht, sodass der gasförmige und/oder dampfförmige Anteil der Creme oder des Gels die transportfähige Form bildet und über den Stoffausgang des Geräts ins Ohrinnere abgegeben wird.

Ist das Wirkstoffpräparat eine Kapsel, wird diese soweit erwärmt, bis sich die Kapselhülle öffnet und der gasförmige und/oder gasförmige Anteil der Füllung aus der Kapselhülle austreten kann und so die transportfähige Form bildet und über den Stoffausgang des Geräts ins Ohrinnere abgegeben wird.

Ist das Wirkstoffpräparat eine Kapsel und die Füllung gasförmig, wird die Kapsel beispielsweise mittels Perforation geöffnet, wodurch die gasförmige Füllung aus der Kapselhülle austreten kann und so die transportfähige Form bildet und über den Stoffausgang des Geräts ins Ohrinnere abgegeben wird. Es kann auch vorgesehen sein, dass ein dem Sieden/Verdampfen/in den gasförmigen Zustand übergehen ähnlicher Vorgang durch Verwendung anderer Technologien erreicht wird wie beispielsweise Verdampfen mittels Ultraschallbestrahlung einer Flüssigkeit etc.

Das Gerät hat ein Gerätegehäuse mittels dessen die Energieversorgung, die Schnittstelle, die Aufnahme, die Aufbereitungseinheit und der Stoffausgang als kompakte am Ohr tragbare Einheit ausgestaltet sind. Das Gerätegehäuse kann weitere Eigenschaften bereitstellen beispielsweise durch Bildung einer Hülle, die wasserdicht, erschütterungsfest, dämpfend etc. ist.

Gemäss einer Ausführungsform des Geräts ist die transportfähige Form gasförmig und/oder dampfförmig und/oder pulverförmig und/oder flüssig.

Ist die transportfähige Form gasförmig enthält diese den Wirkstoff in Form von Gasteilchen, beispielsweise in der Form von Gasmolekülen des Wirkstoffs.

Ist die transportfähige Form dampfförmig ist sie auch gasförmig und enthält den Wirkstoff in Form kleiner Tröpfchen die einen Dampf bilden. Diese Tröpfchen können neben dem Wirkstoff auch noch andere Stoffe wie Lösungsmittel, beispielsweise Wasser, aufweisen. Dann kann der Wirkstoff in diesem/n Lösungsmittel/n gelöst in Form kleiner Tropfen vorliegen.

Ist die transportfähige Form pulverförmig enthält diese feste Pulverteilchen. Diese festen Pulverteilchen weisen dann den Wirkstoff auf oder der Wirkstoff bildet diese Pulverteilchen, beispielsweise wenn der Wirkstoff pulverförmig vorliegt. Die Pulverteilchen können auch als Trägersubstanz dienen, wobei dann die Pulverteilchen den Wirkstoff beispielsweise in adsorbierter Form oder auf sonstige Weise gebundener Form aufweisen.

Es kann vorgesehen sein, dass Pulverteilchen, die als solche ins Ohrinnere abgegeben werden so ausgestaltet sind, dass sie im Ohrinneren resorbiert werden bzw. sich im Wesentlichen rückstandslos auflösen/zersetzen/verflüchtigen.

Ist die transportfähige Form flüssig, dann bildet der Wirkstoff eine Flüssigkeit oder ist in einer solchen gelöst.

Ist die transportfähige Form gasförmig und/oder dampfförmig und/oder pulverförmig kann diese mittels einer Strömung, beispielsweise einer Strömungsbewegung der Luft, vom Gerät ins Ohrinnere abgegeben werden. Diese Strömung kann durch einen Druckunterschied der zwischen dem Ort an dem das aufbereitete Wirkstoffpräparat vorliegt und dem Geräteäusseren herrscht erzeugt werden. Ein solcher Druckunterschied kann beispielsweise aufgrund der Aufbereitung oder bei der Aufbereitung des Wirkstoffpräparats vom Gerät erzeugt werden.

Wird beispielsweise zur Aufbereitung des Wirkstoffpräparats dieses erwärmt, kann aufgrund dieser Erwärmung ein solcher Druckunterschied erzeugt werden. Wird beispielsweise eine Kapsel als Wirkstoffpräparat verwendet, die eine Füllung unter Druck aufweist kann ein solcher Druckunterschied erzeugt werden bei der Freisetzung der Füllung. Ebenso kann vorgesehen sein, dass das Gerät technische Mittel zur Erzeugung eines solchen Druckunterschieds aufweist.

Es kann auch ein Strömungserzeuger beispielsweise als Teil der Aufbereitungseinheit vorgesehen sein. Dieser Strömungserzeuger erzeugt dann die Strömung wodurch der Wirkstoff, der in transportfähiger Form vorliegt, ins Ohrinnere geströmt bzw. geblasen wird. Des Weiteren ermöglicht die Abgabe des Wirkstoffs in gasförmiger und/oder dampfförmiger und/oder pulverförmiger Form ins Ohrinnere, dass der Wirkstoff direkt und gezielt an der Oberfläche bzw. an den Oberflächen des Ohrinneren appliziert wird. Es ist kein Einfüllen einer Flüssigkeit, wie beispielsweise bei der Applikation von Ohrentropfen, ins Ohrinnere nötig. Das Ohrinnere wird also nicht durch eine Flüssigkeit blockiert und somit muss vom Benutzer auch nicht darauf geachtet werden, dass der Wirkstoff nicht wieder aus dem Ohr austritt, wie dies bei der Applikation von Ohrentropfen der Fall sein kann. Ebenso ist nach der Abgabe des Wirkstoffs in gasförmiger und/oder dampfförmiger und/oder pulverförmiger Form ins Ohrinnere, das Ohrinnere wieder frei, sodass die akustische Wahrnehmung durch den abgegebenen Wirkstoff nicht beeinträchtigt ist. Dies im Gegensatz zu beispielsweise Ohrentropfen die über einen längeren Zeitraum das Ohrinnere blockieren.

Gemäss einer Ausführungsform des Geräts weist die Aufbereitungseinheit eine Verdampfvorrichtung auf.

Die Verdampfvorrichtung bringt den Wirkstoff des Wirkstoffpräparats in den dampfförmigen und/oder gasförmigen Zustand wodurch die transportfähige Form gebildet wird. Die Verdampfvorrichtung ermöglicht eine einfache Aufbereitung des Wirkstoffpräparats zur Bereitstellung des Wirkstoffs in einer transportfähigen Form.

Gemäss einer Ausführungsform des Geräts weist die Verdampfvorrichtung eine Wärmequelle auf zur Erwärmung des Wirkstoffpräparats.

Mittels der Wärmequelle kann das aufgenommene Wirkstoffpräparat bis zu einer Temperatur erwärmt werden, bei der der im Wirkstoffpräparat enthaltene Wirkstoff in den gasförmigen Zustand und/oder dampfförmigen Zustand übergeht und/oder sich gegebenenfalls eine Kapselhülle des Wirkstoffpräparats öffnet. Die Nutzung einer Wärmequelle zur Verdampfung ermöglicht eine einfache und mittels der Steuerung automatisch kontrollierbare Aufbereitung des Wirkstoffpräparats zur Bereitstellung des Wirkstoffs in einer transportfähigen Form.

Es kann auch vorgesehen sein, dass andere Technologien zur Überführung des Wirkstoffs in den gasförmigen und/oder dampfförmigen Zustand verwendet werden. Beispielsweise können mittels Bestrahlung durch Ultraschall auch kleinste Tröpfchen aus einer Flüssigkeit erzeugt werden die dann einen Dampf bilden. Dadurch kann ein Dampf bzw. Nebel aus kleinsten Tröpfchen erzeugt werden ohne dass eine Erwärmung des Wirkstoffpräparats wie oben beschrieben erfolgen muss.

Gemäss einer Ausführungsform des Geräts weist die Aufbereitungseinheit eine Perforationsvorrichtung auf zur Perforation des Wirkstoffpräparats.

Mittels der Perforationsvorrichtung kann ein Wirkstoffpräparat perforiert werden, wodurch der Wirkstoff über die Perforierung freigesetzt wird. Ist beispielsweise das Wirkstoffpräparat eine Kapsel mit einer Kapselhülle, kann diese Kapselhülle mittels der Perforationsvorrichtung perforiert werden. Die Füllung der Kapsel wird dann über die Perforation freigesetzt und bildet dann die transportfähige Form. Es kann auch ein Wirkstoffpräparat, das pulverförmig in kompaktierter Form in der Aufnahme vorliegt perforiert werden, sodass eine Vergrösserung der Oberfläche über welche der Wirkstoff in den gasförmigen und/oder dampfförmigen Zustand gebracht wird vergrössert wird. Die Nutzung einer Perforationsvorrichtung zur Aufbereitung ermöglicht eine einfache, energiesparende und mittels der Steuerung automatisch kontrollierbare Aufbereitung des Wirkstoffpräparats zur Bereitstellung des Wirkstoffs in einer transportfähigen Form. Es kann auch vorgesehen sein, dass die Perforationsvorrichtung losgelöst von der Steuerung ausgebildet ist. Dann kann ein Trigger-Mechanismus die Perforation auslösen. Der Trigger-Mechanismus kann beispielsweise mittels eines betätigbaren mechanischen Auslösers erfolgen. Die Perforationsvorrichtung kann auch zusammen mit der Verdampfvorrichtung im Gerät ausgebildet sein.

Gemäss einer Ausführungsform weist das Gerät einen gegen aussen dichten Transportkanal zur Bereitstellung einer Transportverbindung vom Stoffausgang zum Ohrinneren auf.

Der Transportkanal führt den Wirkstoff der in transportfähiger Form vorliegt vom Gerät zum Ohrinneren, sodass der Wirkstoff über den Stoffausgang des Geräts durch den Transportkanal ins Ohrinnere abgegeben wird. Dieser Transportkanal kann im einfachsten Fall auch durch eine entsprechende Ausformung des Stoffausgangs gebildet werden. Dann würde das Gerät zur Verwendung typischerweise in unmittelbarer Nähe des Ohrinneren oder im Ohrinneren platziert. Vorteilhafter scheint es jedoch zu sein, wenn sich der Transportkanal über eine gewisse Länge erstreckt, sodass dieser eine flexible Anbringung des Geräts am Ohr ermöglicht.

Der Transportkanal kann so ausgebildet sein, dass er gegen aussen dicht ist. Der Wirkstoff der durch den Transportkanal zum Ohrinneren geführt wird, wird dadurch bis zur Abgabe des Wirkstoffs ins Ohrinnere gegen aussen abgeschirmt. Dabei kann sich gegen aussen dicht bzw. die Abschirmung auf unterschiedliche Aspekte der transportfähigen Form des Wirkstoffs beziehen wie beispielsweise gegen aussen wasser-/feuchteundurchlässig, gegen aussen Lösungsmittelundurchlässig gegen aussen gasundurchlässig, gegen aussen lichtundurchlässig, gegen aussen wärmeundurchlässig etc. Dadurch kann erreicht werden, dass die transportfähige Form des Wirkstoffs nur in äusserst begrenztem Masse äusseren Einflüssen ausgesetzt wird bevor sie ins Ohrinnere abgegeben wird.

Gemäss einer Ausführungsform weist der Transportkanal ein erstes und ein zweites Ende auf, wobei der Stoffausgang das erste Ende des Transportkanals bildet und das zweite Ende durch eine Abgabevorrichtung zur Abgabe des Wirkstoffs ins Ohrinnere gebildet wird.

Der Stoffausgang kann das erste Ende des Transportkanals bilden indem der Transportkanal fest und gegen aussen dicht mit dem Stoffausgang verbunden ist. Es kann auch vorgesehen sein, dass die Verbindung des Transportkanals mit dem Stoffausgang lösbar ist, sodass der Transportkanal von dem Gerät trennbar ist. Das andere Ende des Transportkanals ist dazu ausgelegt, um zum Ohrinneren hin oder am Eingang zum Ohrinneren platziert zu werden. Die Abgabevorrichtung die das zweite Ende des Transportkanals bildet ist dazu ausgelegt den Wirkstoff über einen Raumbereich verteilt in eine bestimmte Richtung abzugeben. In einer einfachsten Form kann die Abgabevorrichtung durch den Abschluss des Transportkanals gebildet werden. Der genannte Effekt kann aber beispielsweise auch durch eine trichterförmige Ausgestaltung der Abgabevorrichtung erreicht werden. Die Abgabevorrichtung kann auch einen Zerstäuber aufweisen, der beispielsweise, falls die transportfähige Form flüssig ist, die Flüssigkeit zur Abgabe ins Ohrinnere zerstäubt. Die Abgabevorrichtung ermöglicht dadurch eine optimierte, gerichtete und über einen gewissen Raumbereich verteilte Abgabe des Wirkstoffs ins Ohrinnere.

Gemäss einer Ausführungsform des Geräts ist die Abgabevorrichtung elastisch verformbar ausgebildet, sodass diese zumindest teilweise formschlüssig und/oder teilweise reibungsschlüssig im Ohrinneren positionierbar ist.

Durch diese elastische Verformbarkeit der Abgabevorrichtung kann diese und somit das zweite Ende des Transportkanals am Eingang zum Ohrinneren im Wesentlichen positionsfest angebracht werden. Die elastische Verformbarkeit ermöglicht beispielsweise ein Zusammendrücken der Abgabevorrichtung. Die zusammengedrückte Abgabevorrichtung kann dann am Eingang zum Ohrinneren angebracht werden. Aufgrund der elastischen Verformbarkeit strebt die Abgabevorrichtung, falls keine verformenden Kräfte wirken, den ursprünglichen Formzustand an. Dadurch wird die Abgabevorrichtung einen im Eingang zum Ohrinneren geklemmt gehaltenen Zustand erreichen. Die Klemmung erfolgt mittels Reibungsschluss und/oder Formschluss der Abgabevorrichtung mit dem Eingang ins Ohrinnere.

Es kann vorteilhaft sein, die Abgabevorrichtung so auszugestalten, dass der Reibungsschluss und/oder Formschluss nicht vollständig ist im Sinne von, dass der Eingang zum Ohrinneren durch die geklemmt gehaltene Abgabevorrichtung nicht vollständig blockiert wird. Die Abgabevorrichtung kann dann so ausgestaltet sein, dass im geklemmt gehaltenen Zustand eine offene Verbindung vom Ohrinneren nach aussen bestehen bleibt, sodass beispielsweise ein Druckaufbau im Ohrinneren während der Abgabe des Wirkstoffs ins Ohrinnere verhindert wird. Die elastische Verformbarkeit der Abgabevorrichtung ermöglicht somit ein im Wesentlichen positionsfestes Anbringen der Abgabevorrichtung am Eingang ins Ohrinnere, wodurch eine Positionsveränderung der Abgabevorrichtung während der Abgabe des Wirkstoffs ins Ohrinnere verhindert wird. Dies wiederum trägt zur flexiblen Nutzung des Geräts bei, da die Bewegungsfreiheit des Benutzers dadurch kaum eingeschränkt wird.

Gemäss einer Ausführungsform des Geräts kann der Transportkanal eine thermische Abschirmung aufweisen, die den Transportkanal gegen aussen thermisch abschirmt.

Die thermische Abschirmung des Transportkanals gegen aussen hat zum Effekt, dass die transportfähige Form des Wirkstoffs und auch der Transportkanal nur minimale durch äussere Einflüsse bedingte Temperaturänderung erfährt während der Abgabe des Wirkstoffs ins Ohrinnere. Dadurch kann beispielsweise die Kondensation der transportfähigen Form des Wirkstoffs im Transportkanal zumindest minimiert oder gänzlich verhindert werden.

Gemäss einer Ausführungsform des Geräts wird der Transportkanal durch einen flexiblen Transportschlauch gebildet.

Der flexible Transportschlauch kann beispielsweise ein Kunststoffschlauch sein. Ein solcher Kunststoffschlauch kann einfach und kosteneffizient hergestellt werden. Die Verwendung eines Kunststoffschlauchs bietet auch die nötige Flexibilität bezüglich Anforderungen im Zusammenhang mit Hygiene- und Biokompatibilitätsvorschriften. Der Transportschlauch kann aber auch aus einem anderen geeigneten Material gefertigt sein. Es kann vorgesehen sein, dass der Transportschlauch im Sinne eines Verbrauchsartikels regelmässig zu ersetzen ist.

Gemäss einer Ausführungsform des Geräts weist das Gerät einen Strömungserzeuger auf, wobei das Gerät dazu ausgelegt ist, die transportfähige Form des Wirkstoffs mittels der vom Strömungserzeuger erzeugten Strömung über den Transportausgang ins Ohrinnere abzugeben.

Der Strömungserzeuger kann eine Bewegung der Luftmassen, d.h. eine Strömungsbewegung der Luftmassen erzeugen. Alternativ, kann der Strömungserzeuger eine Bewegung einer Flüssigkeit, d.h. eine Strömungsbewegung einer Flüssigkeit erzeugen. Die transportfähige Form des Wirkstoffs, die der so erzeugten Strömung ausgesetzt ist wird mittels der Strömung und dem Transportkanal über den Stoffausgang zum Ohrinneren bewegt und ins Ohrinnere geströmt. Der Strömungserzeuger erzeugt eine Strömung mittels derer Wirkstoffe die in gasförmiger und/oder dampfförmiger und/oder pulverförmiger und/oder flüssiger Form vorliegen zum Ohrinneren bewegbar sind. Es kann vorgesehen sein, dass der Strömungserzeuger selektiv dann eingesetzt wird, falls eine vorhandene Strömung nicht ausreichend ist um den Wirkstoff zum Ohrinneren zu bewegen oder falls die transportfähige Form des Wirkstoffs pulverförmig und/oder flüssig ist und bewegt werden soll. Ein Strömungserzeuger ermöglicht somit eine verstärkte und besser kontrollierbare Abgabe des Wirkstoffs ins Ohrinnere.

Gemäss einer Ausführungsform des Geräts ist der Strömungserzeuger ein Gebläse, das dazu ausgelegt ist die transportfähige Form des Wirkstoffs über den Stoffausgang ins Ohrinnere zu blasen.

Es kann auch vorgesehen sein, dass das Gebläse dazu ausgelegt ist bewegte Luftmassen ohne Wirkstoff ins Ohrinnere zu blasen. Beispielsweise kann ein solcher Vorgang vor oder nach erfolgter Abgabe des Wirkstoffs ins Ohrinnere ein Trocknen des Ohrinneren bewirken. Ein Gebläse stellt somit auch weitere Funktionalitäten für das Gerät bereit.

Gemäss einer Ausführungsform des Geräts stellt die Aufnahme über den Stoffausgang und den Transportkanal eine gegen aussen dichte Transportverbindung zum Ohrinneren bereit.

Dabei ist die Aufnahme selbst ebenfalls dicht gegen aussen und nur zum Stoffausgang hin geöffnet. Dadurch kann der Effekt der zur Erzeugung eines Druckunterschieds genutzt wird verstärkt werden.

Gemäss einer Ausführungsform des Geräts weist das Gerät am Gerätegehäuse eine Eingabevorrichtung auf, die kommunikativ mit der Schnittstelle verbunden ist. Diese kommunikative Verbindung kann beispielsweise auf eine kabelgebundene oder kabellose Art bereitgestellt sein.

Die Eingabevorrichtung kann beispielsweise ein oder mehrere betätigbare Knöpfe umfassen, welche bei Betätigung bestimmte Steueranweisungen an die Schnittstelle geben. Solche Steueranweisungen können beispielsweise die Aufbereitung des Wirkstoffpräparats veranlassen oder auch die Kontrolle anderer mit der Schnittstelle und/oder Steuerung kommunikativ verbundener Einheiten wie beispielsweise einen Strömungserzeuger betreffen. Die Eingabevorrichtung trägt somit auch zur flexiblen Nutzung des Geräts bei, sodass die Bewegungsfreiheit eines Benutzers nicht eingeschränkt wird.

Gemäss einer Ausführungsform des Geräts, weist das Gerät eine Abtasteinheit auf, die dazu ausgelegt ist das Ohrinnere, in das der Wirkstoff abgegeben wird, mittels Abtaststrahlung abzutasten und/oder eine Sensoreinheit, die dazu ausgelegt ist einen Anteil mindestens eines gasförmigen/dampfförmigen Stoffes des Ohrinneren zu bestimmen.

Die Abtasteinheit weist technische Mittel auf zur berührungslosen Abtastung des Ohrinneren mittels geeigneter Abtaststrahlung, beispielsweise zur Abtastung der Oberfläche des Ohrinneren und somit auch des Teils der Oberfläche der durch die Haut gebildet wird. Die Abtaststrahlung kann beispielsweise Licht, Infrarotstrahlung, oder eine beliebige andere Art von Strahlung sein. Die Abtasteinheit kann beispielsweise ein optischer Scanner sein.

Es kann vorgesehen sein, dass die Abtasteinheit beispielsweise mittels Infrarotstrahlung Temperaturdaten bereitstellt zur Bestimmung der Temperatur des Ohrinneren. Es kann vorgesehen sein, dass diese Temperaturdaten die Temperatur an einer Stelle der Oberfläche des Ohrinneren betreffen oder auch, dass die Temperaturdaten mehrere unterschiedliche Stellen der Oberfläche des Ohrinneren betreffen. Beispielsweise kann so eine Temperaturverteilung im Ohrinneren bzw. über die Oberfläche des Ohrinneren ermittelt werden. Es kann auch vorgesehen sein, dass solche Temperaturdaten und/oder eine solche Temperaturverteilung zur Behandlungsunterstützung verwendet wird, beispielsweise für ein "Monitoring" des Behandlungsverlaufs und/oder dahingehend, dass basierend darauf die Abgabe des Wirkstoffs ins Ohrinnere angepasst wird.

Temperaturdaten scheinen dazu geeignet, weil, wie oben beschrieben, die Ursache für Beschwerden im Ohrinneren häufig eine Entzündung ist. Der Teil der Oberfläche des Ohrinneren, welcher eine Entzündung aufweist, beispielsweise entzündete Haut, weist typischerweise eine erhöhte Temperatur auf. Temperaturdaten, die einen entzündeten Teil der Oberfläche betreffen können beispielsweise genutzt werden, um die Temperatur der entzündeten Stelle in einem zeitlichen Verlauf zu ermitteln. Ein solcher zeitlicher Temperaturverlauf kann verwendet werden, um den Heilungsprozess zu verfolgen.

Es kann auch vorgesehen sein, dass die Abtasteinheit eine Abbildungseinheit ist, die dazu ausgelegt ist, das Ohrinnere mittels Abtaststrahlung abzutasten und Abtastdaten zur Erstellung eines Abbilds des Ohrinneren bereitzustellen. Ein solches Abbild kann Informationen zur räumlichen Ausgestaltung des Ohrinneren enthalten. Es kann vorgesehen sein, dass solche Informationen zur räumlichen Ausgestaltung des Ohrinneren verwendet werden, beispielsweise um die Abgabe des Wirkstoffs ins Ohrinnere anzupassen. Es kann vorgesehen sein, dass solche Informationen zur räumlichen Ausgestaltung des Ohrinneren für das "Monitoring" des Behandlungsverlaufs verwendet werden.

Die Sensoreinheit kann beispielsweise mindestens einen Gassensor umfassen. Mittels des mindestens einen Gassensors können Sensordaten bereitgestellt werden zur Bestimmung eines Anteils eines gasförmigen/dampfförmigen Stoffs des Ohrinneren. Ein solcher Gassensor kann beispielsweise ein Feuchtesensor, ein CO2-Sensor, oder ein Gassensor, der gasförmige Kohlenwasserstoffverbindungen detektiert sein. Mittels solcher Sensordaten lässt sich beispielweise eine Zusammensetzung der gasförmigen und/oder dampfförmigen Stoffe (Feuchte, CO2, Lösungsmitteldämpfe, Wirkstoff etc.) im Ohrinneren und somit eine mit der Abgabe des Wirkstoffs ins Ohrinnere in Verbindung stehende Änderung dieser Zusammensetzung ermitteln/beobachten. Es kann vorgesehen sein, dass solche Sensordaten für das "Monitoring" des Behandlungsverlaufs verwendet werden bzw. basierend darauf die Abgabe des Wirkstoffs ins Ohrinnere angepasst wird.

Gemäss einer Ausführungsform des Geräts weist das Gerät eine Lichtquelle zur Bestrahlung des Ohrinneren auf, wobei die Lichtquelle dazu ausgelegt ist, Licht, das eine desinfizierende Wirkung hat, auszustrahlen.

Die Lichtquelle kann beispielsweise eine Lichtquelle sein die ultraviolettes (UV) Licht aussendet. UV Licht hat eine desinfizierende Wirkung und kann Viren, Bakterien und Pilze unschädlich machen. Es kann vorgesehen sein, dass das Gerät dazu ausgelegt ist das Licht das eine desinfizierende Wirkung hat ins Ohrinnere zu strahlen, um das Ohrinnere zu desinfizieren bzw. Viren, Bakterien, Pilze unschädlich zu machen. Es kann vorgesehen sein, dass eine solche Behandlung mit dem Licht präventiv erflogt oder auch im Zusammenhang mit der Behandlung bei der ein Wirkstoff ins Ohrinnere abgegeben wird.

Wie oben beschrieben kann die Steuerung auch nicht Teil des Geräts sein. Beispielsweise kann die Steuerung durch ein mobiles Datenverarbeitungsgerät bereitgestellt werden. Ein solches Gerät kann ein Mobiltelefon, ein Tablet-Computer, ein Laptop oder vergleichbares Gerät sein. Es kann vorgesehen sein, dass mittels einer Softwareapplikation (mobile App bzw. App), die auf dem mobilen Datenverarbeitungsgerät mit der Steuerung ausgeführt wird, dem Benutzer die Möglichkeit bereitgestellt wird das Gerät zu Steuern bzw. die Behandlung mittels entsprechender Daten/Temperaturdaten/Abtastdaten/Informationen zur räumlichen Beschaffenheit des Ohrinneren zu verfolgen/anzupassen.

Gemäss einer Ausführungsform des Geräts weist das Gerät das Wirkstoffpräparat auf.

Gemäss einer Ausführungsform des Geräts liegt das Wirkstoffpräparat als Pulver und/oder Tablette und/oder Flüssigkeit und/oder Dragee und/oder Paste und/oder Creme und/oder Gel und/oder Kapsel mit einer Kapselhülle und einer Füllung vor.

Im Fall, dass das Wirkstoffpräparat als Kapsel ausgebildet ist, ist die Füllung von der Kapselhülle eingekapselt und durch diese gegen aussen abgeschlossen.

Gemäss einer Ausführungsform ist die Füllung der Kapsel flüssig, gasförmig oder pulverförmig.

Die Erfindung betrifft auch die Verwendung eines Wirkstoffpräparats in einem erfindungsgemässen Gerät.

Die Erfindung kann auch ein Kit betreffen, das ein erfindungsgemässes Gerät und eine Desinfektionsvorrichtung aufweist, wobei die Desinfektionsvorrichtung eine UV-Lichtquelle aufweist und dazu ausgelegt ist das Gerät aufzunehmen und mit UV-Licht zur bestrahlen. Es kann auch vorgesehen sein, dass die Desinfektionsvorrichtung ferner dazu ausgelegt ist einen Energiespeicher der Energieversorgung des Geräts aufzuladen.

Im Folgenden wird zur Veranschaulichung ein möglicher Benutzungsvorgang des erfindungsgemässen Geräts beschrieben.

Eine betroffene Person/Ein Benutzer mit einer Entzündung im Ohrinneren bestückt das Gerät mit einem für die Behandlung vorgesehenen Wirkstoffpräparat, versetzt das Gerät in einen betriebsbereiten Zustand und bringt das Gerät am Ohr an. Dann veranlasst der Benutzer die Aufbereitung des Wirkstoffpräparats. Der Wirkstoff wird dann in einer transportfähigen Form ins Ohrinnere und an die zu behandelnde Stelle abgegeben. Nach erfolgter Abgabe des Wirkstoffs kann das Gerät vom Ohr entfernt werden und die Applikation ist abgeschlossen.

### -KURZE BESCHREIBUNG DER FIGUREN-

Das erfindungsgemässe Gerät wird nachfolgend anhand von in den Zeichnungen schematisch dargestellten konkreten Ausführungsbeispielen rein beispielhaft näher beschrieben, wobei auch auf weitere Vorteile der Erfindung eingegangen wird. Im Einzelnen zeigen:
- Figur 1: am Ohr getragene Ausführungsform des Geräts;
- Figur 2: eine Ausführungsform des Geräts mit einem betätigbaren Knopf und einer Abgabevorrichtung;
- Figur 3: eine Ausführungsform des Geräts mit in die Aufnahme eingesetztem Wirkstoffpräparat.

### -DETAILLIERTE BESCHREIBUNG DER ERFINDUNG-

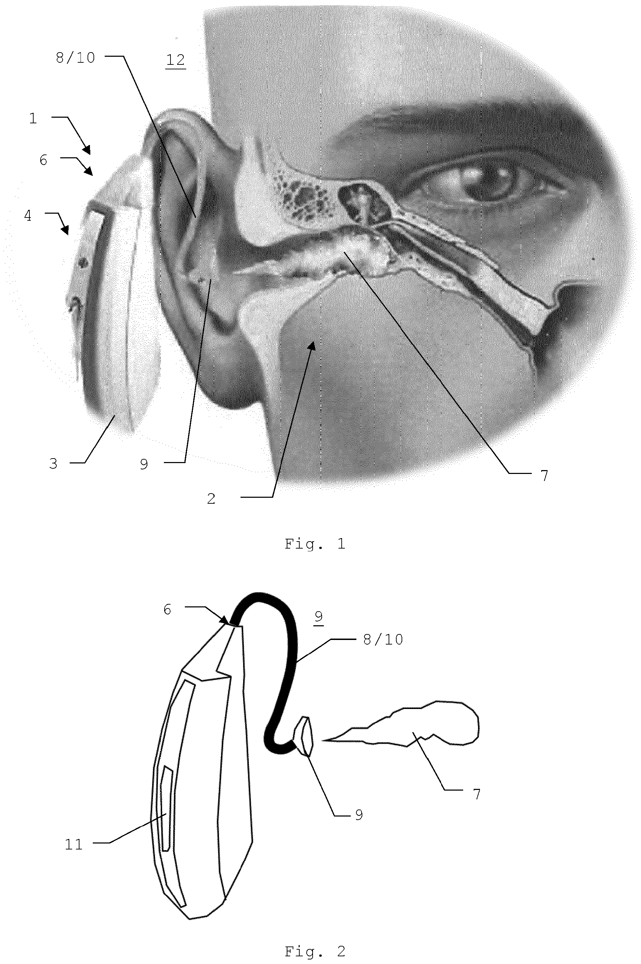

Die Figur 1, zeigt eine Ausführungsform des erfindungsgemässen Geräts 1. Das Gerät 1 weist eine Energieversorgung, eine Schnittstelle zum Empfangen von Steueranweisungen, ein Gerätegehäuse 3, eine Aufnahme 4 für ein Wirkstoffpräparat 5, eine Aufbereitungseinheit, und einen Stoffausgang 6 auf. Die Energieversorgung, die Schnittstelle, die Aufnahme 4, das Wirkstoffpräparat 5, die Aufbereitungseinheit und der Stoffausgang 6 sind nicht gezeigt, da sie durch das Gerätegehäuse verdeckt sind. Das Gerät ist am Ohr angebracht und weist einen Transportkanal 8 in Form eines flexiblen Transportschlauchs 10 auf. Das eine Ende des Transportkanals wird von dem Stoffausgang 6 gebildet, das andere Ende des Transportkanals wird von der trichterförmigen Abgabevorrichtung 9 gebildet. Die Abgabevorrichtung ist vor dem Ohrinneren angebracht, sodass der Wirkstoff in seiner transportfähigen Form 7 ins Ohrinnere abgegeben werden kann. In Figur 1 ist die Abgabe des Wirkstoffs in Form eines Dampfs/Nebels ins Ohrinnere gezeigt. Es kann auch vorgesehen sein, dass die Abgabevorrichtung 9 einen Zerstäuber aufweist. Dann kann der Wirkstoff die Abgabevorrichtung in flüssiger Form erreichen und wird dort mittels des Zerstäubers zu einem Nebel zerstäubt und als Nebel ins Ohrinnere abgegeben.

Figur 2 zeigt schematisch eine Ausführungsform des erfindungsgemässen Geräts 1. Das Gerät hat eine Eingabevorrichtung die als Kippschalter 11 ausgebildet ist. Der Kippschalter kann eine Ein- und eine Aus-Stellung einnehmen. Befindet sich das Gerät 1 in einem Betriebsbereiten Zustand, d.h. es befindet sich ein Wirkstoffpräparat in der Aufnahme, dann ist der Aufbereitungsvorgang dadurch veranlassbar, dass der Kippschalter in die Ein-Stellung gebracht wird.

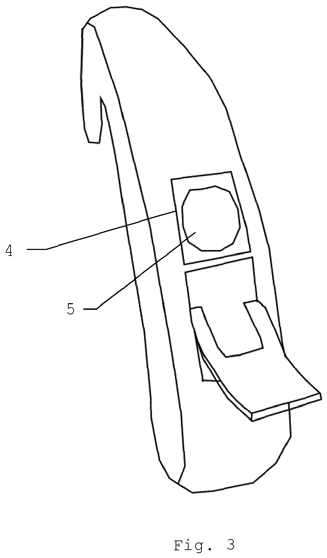

Figur 3 zeigt schematisch eine Ausführungsform des Geräts 1 mit einer Aufnahme 4 für ein Wirkstoffpräparat 5. Die Aufnahme 4 weist eine verschliessbare Öffnung auf, durch welche das Wirkstoffpräparat 5 in die Aufnahme 4 eingesetzt werden kann. Das Wirkstoffpräparat 5 kann unterschiedlich ausgebildet sein.

Das Wirkstoffpräparat kann eine Kapsel sein mit einer Kapselhülle und einer Füllung. Die Kapselhülle besteht typischerweise aus Gelatine, Cellulose, Carrageen, oder Stärke. Die Kapselhülle kann aber auch aus anderen für die Herstellung von medizinischen Kapselhüllen typischerweise verwendeten Stoffe bestehen. Die Füllung umfasst den Wirkstoff und kann beispielsweise zusätzlich oder als Wirkstoff Stoffe einer Zitrone, Zwiebel, von Knoblauch, Salbei, Minze, Kamille etc. umfassen. Es können Kapseln für das Gerät vorgesehen sein, welche sich bei einer Temperatur von ca. 40° so verändern, dass die Füllung freigegeben wird. Dazu kann die Kapselhülle bei der Temperatur beispielsweise schmelzen oder sich zersetzen.

Es können die unterschiedlichsten Arten von Kapseln mit dem Gerät verwendet werden. Beispielsweise Hartkapseln, Weichkapseln, Mikrokapseln, Cellulosekapseln, Stärkekapseln, etc. welche nach den gängigen Verfahren hergestellt werden.

Dies ermöglicht eine kosteneffiziente Bereitstellung einer Vielzahl unterschiedlicher Kapseln, welche auch unterschiedliche Wirkstoffe enthalten. Dadurch ermöglicht das erfindungsgemässe Gerät die Applikation einer Vielzahl an Wirkstoffen, wodurch eine Vielzahl an spezifischen Behandlungen des Ohrinneren für einen Betroffenen in Absprache mit einem Spezialisten durchführbar sind.

Es versteht sich, dass diese dargestellten Figuren nur mögliche Ausführungsbeispiele schematisch darstellen.

## Patentansprüche

1. Gerät (1), am oder im Ohr tragbar und zur Abgabe eines Wirkstoffs ins Ohrinnere (2) ausgelegt, aufweisend
o eine Energieversorgung,
o eine Schnittstelle zum Empfangen von Steueranweisungen
o ein Gerätegehäuse (3),
o eine Aufnahme (4) für ein Wirkstoffpräparat (5),
o eine Aufbereitungseinheit, und
o einen Stoffausgang (6),
wobei
o die Aufbereitungseinheit mittels der Schnittstelle steuerbar ist und dazu ausgelegt ist, ein Wirkstoffpräparat (5) so aufzubereiten, dass der Wirkstoff in transportfähiger Form (7) vorliegt und über den Stoffausgang (6) ins Ohrinnere (2) abgebbar ist, und
o die Energieversorgung, die Schnittstelle, die Aufnahme (4), die Aufbereitungseinheit und der Stoffausgang (6) mittels des Gerätegehäuses (3) eine Einheit bilden.

2. Gerät nach Anspruch 1, **dadurch gekennzeichnet, dass** die transportfähige Form (7) gasförmig und/oder dampfförmig und/oder pulverförmig und/oder flüssig ist.

3. Gerät nach einem der Ansprüche 1 bis 2, **dadurch gekennzeichnet, dass** die Aufbereitungseinheit eine Verdampfvorrichtung aufweist.

4. Gerät nach Anspruch 3, **dadurch gekennzeichnet, dass** die Verdampfvorrichtung eine Wärmequelle aufweist zur Erwärmung des Wirkstoffpräparats (5).

5. Gerät nach einem der Ansprüche 1 bis 4, **dadurch gekennzeichnet, dass** die Aufbereitungseinheit eine Perforationsvorrichtung aufweist zur Perforation des Wirkstoffpräparats.

6. Gerät nach einem der Ansprüche 1 bis 5, **gekennzeichnet durch** einen gegen aussen (12) dichten Transportkanal (8) zur Bereitstellung einer Transportverbindung vom Stoffausgang (6) zum Ohrinneren (2).

7. Gerät nach Anspruch 6, **dadurch gekennzeichnet, dass** der Transportkanal (8) ein erstes und ein zweites Ende aufweist, wobei der Stoffausgang (6) das erste Ende des Transportkanals (8) bildet und das zweite Ende durch eine Abgabevorrichtung (9) zur Abgabe des Wirkstoffs (7) ins Ohrinnere (2) gebildet wird.

8. Gerät nach Anspruch 7, **dadurch gekennzeichnet, dass** die Abgabevorrichtung elastisch verformbar ausgebildet ist, sodass diese zumindest teilweise formschlüssig und/oder teilweise reibungsschlüssig im Ohrinneren (2) positionierbar ist.

9. Gerät nach einem der Ansprüche 6 bis 8, **dadurch gekennzeichnet, dass** der Transportkanal (8) eine thermische Abschirmung aufweist, die den Transportkanal (8) gegen aussen (12) thermisch abschirmt.

10. Gerät nach einem der Ansprüche 6 bis 9, **dadurch gekennzeichnet, dass** der Transportkanal (8) durch einen flexiblen Transportschlauch (10) gebildet wird.

11. Gerät nach einem der Ansprüche 1 bis 10, **gekennzeichnet durch** ein Strömungserzeuger, wobei das Gerät dazu ausgelegt ist die transportfähige Form des Wirkstoffs (7) mittels der vom Strömungserzeuger erzeugten Strömung über den Stoffausgang (6) ins Ohrinnere (2) abzugeben.

12. Gerät nach Anspruch 11, **dadurch gekennzeichnet, dass** der Strömungserzeuger ein Gebläse ist, das dazu ausgelegt ist die transportfähige Form des Wirkstoffs (7) über den Stoffausgang (6) ins Ohrinnere (2) zu blasen.

13. Gerät nach einem der Ansprüche 1 bis 12, **dadurch gekennzeichnet, dass** die Aufnahme (4) über den Stoffausgang (6) und den Transportkanal (8) eine gegen aussen (12) dichte Transportverbindung zum Ohrinneren (2) bereitstellt,
und/oder
dadurch, dass das Gerät am Gerätegehäuse (3) eine Eingabevorrichtung (11) aufweist, die kommunikativ mit der Schnittstelle verbunden ist,
und/oder
durch eine Abtasteinheit, die dazu ausgelegt ist das Ohrinnere, in das der Wirkstoff abgegeben wird, mittels Abtaststrahlung abzutasten,
und/oder
durch eine Sensoreinheit, die dazu ausgelegt ist einen Anteil mindestens eines gasförmigen/dampfförmigen Stoffes des Ohrinneren zu bestimmen,
und/oder
durch eine Lichtquelle zur Bestrahlung des Ohrinneren, wobei die Lichtquelle dazu ausgelegt ist, Licht, das eine desinfizierende Wirkung hat, auszustrahlen.

14. Gerät nach einem der Ansprüche 1 bis 13, **dadurch gekennzeichnet, dass** das Gerät ein Wirkstoffpräparat (5) aufweist,
und/oder
dadurch, dass das Wirkstoffpräparat (5) als Pulver und/oder Tablette und/oder Flüssigkeit und/oder Dragee und/oder Paste und/oder Creme und/oder Gel und/oder Kapsel mit einer Kapselhülle und einer Füllung vorliegt,
und/oder
dadurch, dass die Füllung flüssig, gasförmig oder pulverförmig ist.

15. Verwendung eines, insbesondere als Kapsel ausgebildeten, Wirkstoffpräparats (5), in einem Gerät nach einem der Ansprüche 1 bis 14.
